# EUROPEAN PATENT SPECIFICATION

(11) **EP 0 638 304 B1**
(45) Date of publication and mention of the grant of the patent: **10.03.1999**
(21) Application number: 93202087.8
(22) Date of filing: 16.07.1993
(51) Int. Cl.: A61F 13/64

(54) **Pant style diapers**
Höschenartige Windeln
Couches culottes du type slip

(43) Date of publication of application: 15.02.1995
(73) Proprietor: THE PROCTER & GAMBLE COMPANY, Cincinnati, Ohio 45202 (US)
(72) Inventor: Schmitz, Christoph Johann, D-53881 Euskirchen (DE)
(74) Representative: Canonici, Jean-Jacques

(56) References cited:
- EP-A- 0 352 208
- FR-A- 2 586 558
- US-A- 1 580 464
- US-A- 4 205 679

## Description

### Field of the invention

The present invention relates to disposable absorbent articles or undergarments. The disposable articles are essentially designed as conventional disposable diapers however they are provided with an endless circular waistband at one longitudinal end of the article. The disposable article is applied to a wearer by slipping the endless waistband onto the hips such that the absorbent part of the article is in front or behind the wearer and can be pulled between the legs to the opposing side and be attached to the endless waistband.

### Background of the invention

Undergarments and disposable diapers are well established products. The prior art on belts or bands in connection with disposable diapers is also very crowded. For example US 849725 and US 3890973 disclose absorbent garments having two belts which in use form an endless circular belt together with the front and back longitudinal end of the absorbent product. In its flat, stretched out shape this product has one part of an elastic belt laterally extending from each side of the garment.

US 1,580,464 disclose a bifurcated garment adapted to encircle the hips and upper leg portions of the body. The patent teaches the preamble of claim 1 of the present invention.

US 3618608 and 4128326 as well as FR 2586558 disclose diapers having a belt adjacent or near one of the waist margins. The belt laterally extends to either side of the diaper and is attached to the opposite waist margin. US 4158906 discloses a diaper such which fastens opposing sides of the diaper. The length of the diaper sinch is adjustable and is independent from the rest of the diaper. US 4315508 discloses garments having two elastic pieces for form a removable elastic belt. One piece on each side connects the front and the rear waist margins of the garment. FR 2612770 discloses disposable pants having integral straps which encircle the waist of the wearer and are secured by fasteners.

EP-A-409307 or PCT 9007160 both disclose attachable elastic waist belts and EP-A-352208 and DE-A-3238450 disclose disposable diapers having a detachable elastic waist belt which allows them to be used like pull-up pants or regular undergarments. US 4205679 also discloses disposable undergarments which can be slipped on like regular underpants having an elastic waist band. A leak proof underpants carrying a disposable catamenial is known from US 4690681 which allows the wearer to put the catamenial on in the same fashion as a regular underpants. A similar construction used as a child's training pants is known from US 4646362 also having an elasticized waist band and elasticized leg openings.

One basic problem with undergarment or underpants type disposable garments, is that in order to use them the wearer has to slip them on over the legs which in particular for young children can be difficult due to their activity level. Also they often do not wish to wear such disposable absorbent articles and physically reject being dressed with them. Therefore it would be easier to just slip a belt over the head or from below over the legs onto the hip of a child rather than trying to put both legs into separated leg opening of an undergarment.

A problem with flat structured disposable articles for example diapers or children's training pants is that in order to apply them onto the wearer the wearer has to be in a lying position and allow tight fitting. Otherwise the functionality of the absorbent article will suffer.

Therefore it is an objective of the present invention to provide a disposable absorbent article which combines the benefits of a flat structured closable absorbent article while not requiring the wearer to lie down and accept manipulating of the article while the wearer has to hold still.

Further in its particular designs this invention also allows a functional decoupling between the absorbent part of the article and the elastic waist band. In particular the draw-back of providing elasticity in the back of a disposable undergarment typically used in the prior art the present invention allows to provide elasticity where it is needed most i.e. around the belly and the hips of a wearer.

Further features and advantages will become evident from the detailed description of the invention and the drawings described below.

### Brief description of the invention

The present invention relates to a disposable absorbent article such as claimed in claim 1.

It is particularly preferred if the oblong substrate is joined in the lateral side regions to the article such that an attachment is formed in longitudinal direction and coincides with the lateral outside edge of the article.

Even more preferred the oblong substrate is disposed in the longitudinal first end region of the article. Typically and preferred the first longitudinal end region is the rear end region of the disposable article. This means that during use on a wearer that first longitudinal end region is in the back of the wearer.

The elastic region in the endless circular waist band is preferably provided by the oblong substrate which in particular can be an elastic laminate of non-woven fibrous substrates.

In use after having placed the waist band around the waist of the wearer the second longitudinal end region is pulled between the legs of the wearer and joined to the oblong substrate so as to form an undergarment on the body of the wearer. Joining the second longitudinal end region to the oblong substrate should preferably be done by a releasable closure system like for example an adhesive tape or a mechanical closure means in particular of the hook/loop type (e.g. VELCRO ™). The joining of the second longitudinal end region to the oblong substrate can also be in such a way that the second longitudinal end region is pulled between the legs of the wearer and through the circular waist band such that the elasticity of the waist band holds the second longitudinal end region pressed on the wearer and is thereby joined with or without particular attachment features to the second longitudinal end region. This may preferable be accomplished in combination with a releasable closure system as indicated above.

Other particular embodiments of the invention are set out in claims 3,4, 8-14.

### Brief description of the drawings

Figure 1 is a plan view of a preferred embodiment of the present invention.
Figure 2 is a perspective view of an alternative embodiment of the present invention.
Figure 3 is a plan view of the disposable absorbent article in yet another alternative embodiment of the present invention when used by a wearer.

### Detailed description of the invention

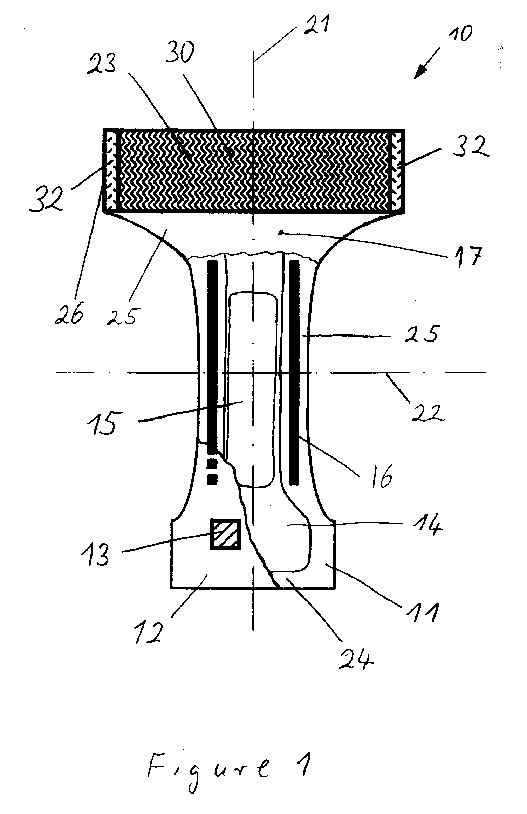

In the following reference will be made to the preferred embodiments of the present invention as shown in Figures 1-3. The present invention is however not limited to the preferred embodiments but defined by the claims following this description.

The term "absorbent article" in the following description refers to absorbent articles including adult incontinence products, female hygiene pads or catamenials, baby diapers and children's training pants.

The term "elastic" refers to a stretchable and self-retractive material characteristic found in natural and artificial rubbers, elastomers and polymers.

Figure 1 shows a preferred embodiment of a disposable absorbent article, namely a training diaper (10). The training diaper (10) has a longitudinal direction (21) defining a first longitudinal end region (23) and a second longitudinal end region (24). It also has a lateral direction (22) defining the lateral side regions (25).

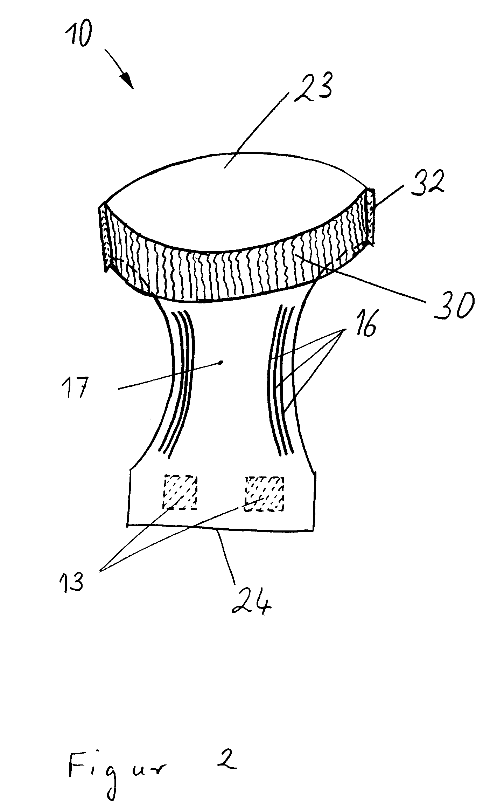

The disposable absorbent training pant (10) typically is structured like a regular disposable diaper portion (17). Diaper portion (17) comprises a top sheet (12) a backsheet (11) and an absorbent core (14) together with an additional core patch (15). In the figures preferred embodiments having single (figure 1) or multiple (figure 2) straight or shaped leg elastics (16) are shown.

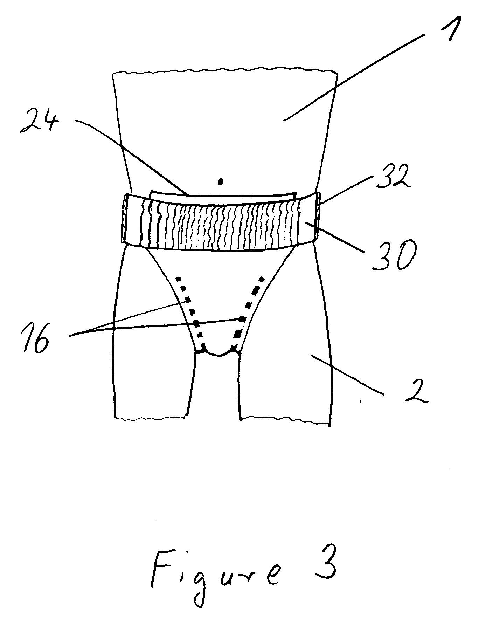

In it first longitudinal end region the disposable absorbent article comprises an oblong substrate (30) which is extending into both lateral side regions (25). The oblong substrate (30) is joined to the training diaper (10) at the lateral side edges (25) as shown in the figures. It is joined by a bond (32) extending a short distance in lateral direction towards the longitudinal axis (21). Depending on the bond strength of the attachment the bond (32) typically has a lateral extension of 3 mm to 30 mm, preferably 5 mm to 15 mm. It is typically as long in longitudinal direction as the oblong substrate (30). The oblong substrate (30) together with the diaper portion (17) forms an endless circular waste band in the first longitudinal end region (23). The endless circular waste band formed by the oblong substrate in the first longitudinal end region comprises an elastic region providing at least tangential elasticity to the circular waste band. In the figures this elastic region is shown as part of the oblong substrate (30). In Figure 1 and 2 the oblong substrate (30) and the elastic region of the endless circular waste band are coextensive while in Figure 3 only a central part of the oblong substrate (30) is elastic. The elastic region can however also be part of the absorbent diapers portion forming the other part of the endless circular waste band. As can be seen from figure 3 the preferred construction of the disposable absorbent article of the present invention is worn such that the first longitudinal end region (23) is in the rear of the wearer (1) while the second longitudinal end region (24) after being pulled between the legs of the wearer (1) is joined to the oblong substrate (30) in the front of the wearer (1).

In order to fit the disposable article (10) onto a wearer (1) as shown in figure 3 several ways to join the oblong substrate (30) and the second longitudinal end region (24) to each other can be employed. It is preferred that releasable cooperating closure means are used, for example adhesive tapes on the oblong substrate or in the second longitudinal end region of the diaper portion (17) can be joined to the respective countersurfaces. Alternatively and preferably mechanical cooperating closure means in particular hook/loop entanglement type systems can be used.

A particularly preferred design is shown in figure 2 and 3 where hook patch (13) cooperating with loops (not shown in the figures) are disposed on the outside of the backsheet (11) of the training diaper. The endless circular waste band is slipped over the legs and hip of a wearer (1) as shown in figure 3 and the second longitudinal end region is pulled between the legs of the wearer and through the inside of the endless circular waste band such that the hook patches (13) are covered by the oblong substrate (30). The oblong substrate (30), in this case preferably on its inside, has a soft feeling compliant loop material providing the counter mechanical closure system surface for the hook patches (13). The opposite type of closure is indicated in figure 1 where hook patches (13) are disposed on the topsheet of the training diaper (10) such that when used according to the above described procedure they have to entangle with the outside of the oblong substrate (30) and lack the benefit of being tightened addionally by the elastic circular tangential force of the endless circular waste band against the wearer (1).

It will be apparent to those skilled in the art that many combinations of materials and design modification are possible when using the beneficial principle of the present invention to combine a flat disposable absorbent article with an elastic endless circular waste band. In the following description materials useful in providing disposable absorbent articles according to the invention, especially training diapers are summarized by way of non-limiting examples.

The absorbent core (14) as well as the optional additional core patch (15) may be any absorbent means which is generally compressible, conformable non-irritating to the wearer's skin and capable of absorbing and retaining liquid such as urine and other certain body exudates. The absorbent core may be manufactured in a wide variety of sizes and shapes and from a wide variety of liquid absorbent materials commonly used in diapers and other disposable absorbent articles such as comminuted woodpulp, generally referred to as airfelt, craped cellulose wadding, meltblown polymers including coformed, crosslinked cellulose fibres, tissues including tissue wraps and tissue laminates, absorbent foams, absorbent sponges, super absorbent polymers, absorbent gelling materials or any other equivalent materials or combination of materials. Also the configuration and construction of the absorbent core may be varied. For example the absorbent material may have varying caliper zones, a hydrophillic gradient, a super absorbent gradient, lower average density and lower average basis weight acquisition zones. It also may comprise one or more layers or substrates as shown in figure 1. The total absorbent capacity of the absorbent article should be compatible with design exudate loading and the intended use of the absorbent article.

Also the size and shape of the absorbent core should be varied to accommodate the expected wearer's ranging from infants to adults. Preferred absorbent core designs have been disclosed in US patents 4,610,678; 4,673,402; 4,888,231; 4,834,735; 4, 685,915; 4,781,710; 4,600,458. The preferred absorbent core design provides a crosslinked cellulose patch (15) essentially without super absorbent on top of a mixture of airfelt plus super absorbent polymer and comprising more than 30% super absorbent polymer in the absorbent core (14) as described in EP-A-512010.

The backsheet is impervious to liquids (e.g. urine) and can be provided as a single sheet or as a laminate of two or more layers. The liquid imperviousness of the backsheet can for example be provided by an impervious layer which is preferably manufactured from a thin plastic film, preferably a thermoplastic film, although other flexible liquid impervious materials may be used. Whether the backsheet consists of a single or several layers, it should be flexible. As used herein, the term "flexible" refers to materials which are compliant and which will readily conform to the general shape and contours of the human body. The backsheet prevents the exudates absorbed and contained in the absorbent core from soiling articles which contact the absorbent article such as clothes, bedsheets and undergarments. The backsheet may thus comprise polymeric films such as thermoplastic films of polyethylene or polypropylene, or composite materials such as a film-coated nonwoven material. For economic, aesthetic, and ecological reasons, the part of the backsheet (30) providing liquid imperviousness preferably has an average nominal caliper, i.e. calculated caliper, of less than about 0.051 mm, more preferably a calculated caliper of from 0.01 mm to 0.036 mm.

If provided as a multilayered laminate the backsheet preferably comprises a flexible polyethylene film. As used herein the term "polyethylene" film refers to films which are essentially made of polyethylene, however, it is understood that polyethylene film will contain a variety for additives to provide characteristics like opacity, strength requirements, color or any other desired characteristic that can be achieved through adding minor amounts of other substances than polyethylene into the films. The total amount of additives should be less than 45%, preferably less than 15%, by weight of film materials. Exemplary films for use in backsheets are manufactured by Tredegar Industries, Inc. of Terre Haute, Indiana USA or BP-Chemical PlasTec Rotbuchenstrasse 1, D - 8000 MUNCHEN, Germany.

If the backsheet is provided as a multilayered laminate the outside surface preferably is provide by a layer of material as described below for topsheets. Alternatively the backsheet can also be textured to provide a fibrous outside surface for more clothlike appearance, Further the backsheet may also permit vapors to escape from the absorbent core while still preventing exudates from passing through as described, for example, in US patent 4,681,793. The backsheet may also be biodegradable such as the film disclosed in US Patent Application Serial N0 07/721,066 entitled "Disposable Absorbent Articles with Biodegradable Backsheets".

The size of the backsheet is dictated by the size of the absorbent core and the exact absorbent article design selected. In the preferred embodiment, the backsheet has a modified hourglass shape extending beyond the absorbent core a minimum distance of at least 1.3 cm for adult incontinent briefs around the entire periphery.

The topsheet of the present invention is compliant, soft feeling and non-irritating to the wearer's skin. Further, the topsheet is liquid pervious permitting liquids (e.g. urine) to readily penetrate through its thickness. A suitable topsheet may be manufactured from a wide range of materials, such as porous foams, reticulated foams, apertured films; or woven or nonwoven webs of natural fibers (e.g. wood or cotton fibers), synthetic fibers (e.g. polyester or polypropylene fibers) or from a combination or natural and synthetic fibers. Preferably, it is made of a material that isolates the wearer's skin from liquids retained in the absorbent core.

There are a number of manufacturing techniques which may be used to manufacture the topsheet. For example, the topsheet comprises a nonwoven web of fibers. When the topsheet comprises a nonwoven web, the web may be spunbonded, carded, wet-laid, meltblown hydroentangled, hydroformed, combinations of the above, or the like. An exemplary topsheet is carded and thermally bonded by means well known to those skilled in the fabric art and comprises staple length polypropylene fibers having a denier of about 2.2 and a basis weight from about 15 to about 30 grams per square meter. As used herein the term "staple length fibers" refers to those fibers having a length of at least about 16 mm. A suitable topsheet is manufactured for example by Veratec, Inc. a Division of International paper Company, of Walpole, Massachusetts under the designation P-8. A topsheet particularly preferred for incontinent briefs of the present invention, comprises a formed thermoplastic film such as that described in US Patent 3,929,135 entitled " Absorptive Substrate Having tapered Capillaries".

The topsheet and the backsheet are joined together in any suitable manner as is well known in the diaper manufacturing art. As used herein, the term "joined" encompasses configurations whereby the topsheet is directly joined to the backsheet by affixing the topsheet directly to the backsheet, and configurations whereby the topsheet is indirectly joined to the backsheet by affixing the topsheet to intermediate members (e.g. absorbent core ) which in turn are affixed to the backsheet. In a preferred embodiment, the topsheet and the backsheet are joined directly to each other in the diaper periphery by a flap attachment means such as an adhesive or any other attachment means as is known in the art and indirectly joined in the area coextensive with the absorbent core. In general, the same means as the flap attachment means that affixes the topsheet to the backsheet can also be used as a core attachment means that affixes the absorbent core to the backsheet. Thus, for example, a uniform continuos layer of adhesive, a patterned layer of adhesive, an array of separate lines, spirals, or spots of adhesive such as a network of adhesive filaments such as shown in US Patent 4,573,986, may be used.

The oblong substrate forming part of the endless circular waste band is preferably made such that it has a smooth fabric like outside surface. In particular nonwoven fabric laminates with elastic materials are contemplated as oblong substrates useful in the present invention. The elasticity can be provided before or after the oblong substrate is joined to the diaper portion. If the elasticity is provided after the oblong substrate (30) is joined to the diaper portion (17), which is preferred for manufacturing reasons this can be done by thermal or mechanical activation. Thermal activation uses radiative or convective heating of a nonwoven laminate substrate of which one layer comprises a polymer which shrinks in one or both directions at elevated temperature such that the laminate as a whole contracts. This laminate will allow elastic deformation from its retracted dimension to its original dimension.

Similar but even preferred is when the activation is provided by mechanically stretching, the circular waist band which is to be elasticated at least during a short period. This can be done by use of so called ring rolling which is well known in the art. The region to be elasticated comprises at least one elastic layer of e.g. natural or artificial rubber type polymer and additional layers of polymer film or nonwovens. In the ring rolling process the additional non-elastic layers are permanently stretched while the elastic layer is elastically extended. The elastic layer preferably is sandwiched between the non elastic layers and this laminate is joined in discreet places. Hereby after being ring rolled the laminate provides an elastic region of no strain in its relaxed state. Maximum elongation is equivalent to the activation stretch length during ring rolling. Alternatively, mesh type elastic substrates e.g. with hydro entangled nonwoven fabrics, so called scrim material can be used to provide an oblong substrate particularly useful in the present invention.

A preferred characteristic of the material of the oblong substrate should be the ability to form autogenous bonds in combination with other thermo plastic material or as a combination of identical thermo plastic material to join the oblong substrate to the diaper portion. When using autogenous bonding to join the oblong substrate to the diaper portion instead of adhesive bonding, it is preferred to employ ultrasonic or deformation welding and preferably crimping (high speed deformating welding)

As indicated above the oblong substrate can be an elastic laminate comprising a nonwoven fibrous substrate such that the nonwoven fibres provide the loop surface of the releasable closure system in combination with hook patches discussed above. The elasticity should be such that it provides a comfortable but sufficiently stable placement on a wearer.

## Claims

1. Disposable absorbent article (10) having a longitudinal direction (21) which defines first and second longitudinal end regions (23, 24) and a lateral direction (22) which defines lateral side regions (25) and said disposable absorbent article (10) comprising
an oblong substrate (30),
an endless circular waistband comprising an elastic region,
said oblong substrate (30) being joined to said diaper portion (17) in each said lateral side region (25) such that said oblong substrate (30) together with said diaper portion (17) forms said endless circular waistband;
said elastic region providing at least tangential elasticity to said endless circular waistband,
characterised in that
said oblong substrate (30) is disposed in said lateral direction (22) in said first longitudinal end region (23);
said oblong substrate (30) extending into both said lateral side regions (25) of said diaper portion (17) and said oblong substrate (30) being joined to said disposable absorbent article (10) by an adhesive or autogenous bond of 3 mm to 30 mm width in said lateral direction (22), preferably 5 mm to 15 mm in said lateral direction (22).

2. Disposable absorbent article (10) according to claim 1 such that said oblong substrate (30) is joined to said disposable absorbent article (10) along said lateral outside edge (25) in said longitudinal direction (21).

3. A disposable absorbent article (10) according to any of the preceding claims such that said autogenous bond is formed by ultrasonic or deformation welding, preferably by crimping.

4. A disposable absorbent article (10) according to any of the previous claims such that said oblong substrate (30) is disposed along the outside edge in longitudinal direction (21) of said first longitudinal end region (23) of said disposable absorbent article (10).

5. Disposable absorbent article (10) according to any of the previous claims such that said first longitudinal end region (23) is the rear end region of said disposable absorbent article (10).

6. A disposable absorbent article (10) according to any of the previous claims such that said oblong substrate (30) comprises said elastic region of said endless circular waistband.

7. A disposable absorbent article (10) according to any of the previous claims such that said oblong substrate is an elastic laminate comprising a non-woven fibrous substrate.

8. A disposable absorbent article (10) according to any of the previous claims such that said elastic region of said endless circular waistband is activated by thermal or mechanical action after said endless circular waistband has been formed.

9. A disposable absorbent article (10) according to any of the previous claims such that said disposable absorbent article (10) further comprises cooperating closure means allowing to releasably join said oblong substrate (30) to the second longitudinal end region (24).

10. A disposable absorbent article (10) according to claim 9 such that at least one of said cooperating closure means comprises an adhesive tape.

11. A disposable absorbent article (10) according to claim 9 such that said cooperating closure means comprise mechanical attachment means.

12. A disposable absorbent article (10) according to claim 11 such that said mechanical attachment means are of the hook-loop entanglement type.

13. A disposable absorbent article (10) according to any of the claims 9 to 12 such that at least one of said cooperating closure means is provided on the inside of said endless circular waistband.

14. A disposable absorbent article (10) according to any of the previous claims such that said disposable absorbent article (10) is a diaper (17) comprising a liquid pervious topsheet (12), a liquid impervious backsheet (11) joined to said topsheet (12) and an absorbent core (14) disposed between said topsheet (12) and said backsheet (11).

## Patentansprüche

1. Wegwerfbarer absorbierender Artikel (10) mit einer Längsrichtung (21), welche erste und zweite Längsendbereiche (23, 24) definiert, und einer Querrichtung (22), welche Querseitenbereiche (25) definiert, und wobei der genannte wegwerfbare absorbierende Artikel (10) umfaßt
ein längliches Substrat (30),
ein kreisförmiges Endlos-Taillenband, welches einen elastischen Bereich umfaßt,
wobei das genannte längliche Substrat (30) in jedem genannten Querseitenbereich (25) mit einem Windelabschnitt (17) verbunden ist, sodaß das genannte längliche Substrat (30) zusammen mit dem genannten Windelabschnitt (17) das genannte kreisförmige Endlos-Taillenband bildet;
wobei der genannte elastische Bereich dem genannten kreisförmigen Endlos-Taillenband mindestens tangentiale Elastizität verleiht,
dadurch gekennzeichnet, daß das genannte längliche Substrat (30) in der genannten Querrichtung (22) im genannten ersten Längsendbereich (23) angeordnet ist;
das genannte längliche Substrat (30) sich in beide genannten Querseitenbereiche (25) des genannten Windelabschnitts (17) erstreckt und das genannte längliche Substrat (30) mit dem genannten wegwerfbaren absorbierenden Artikel (10) durch einen Klebstoff oder Autogenbindung von 3 mm bis 30 mm Breite in der genannten Querrichtung (22), vorzugsweise 5 mm bis 15 mm in der genannten Querrichtung (22), verbunden ist.

2. Wegwerfbarer absorbierender Artikel (10) nach Anspruch 1 von der Art, daß das genannte längliche Substrat (30) mit dem genannten wegwerfbaren absorbierenden Artikel (10) entlang dem genannten Seitenaußenrand (25) in der genannten Längsrichtung (21) verbunden ist.

3. Ein wegwerfbarer absorbierender Artikel (10) nach einem der vorhergehenden Ansprüche von der Art, daß die genannte Autogenbindung durch Ultraschall- oder Verformungsschweißen, vorzugsweise durch Kräuselung, gebildet ist.

4. Ein wegwerfbarer absorbierender Artikel (10) nach einem der vorhergehenden Ansprüche von der Art, daß das genannte längliche Substrat (30) entlang dem Außenrand in Längsrichtung (21) des genannten ersten Längsbereichcs (23) des genannten wegwerfbaren absorbierenden Artikels (10) angeordnet ist.

5. Wegwerfbarer absorbierender Artikel (10) nach irgend einem der vorhergehenden Ansprüche von der Art, daß der genannte erste Längsendbereich (23) der hintere Endbereich des genannten absorbierenden Artikels (10) ist.

6. Ein wegwerfbarer absorbierender Artikel (19) nach einem der vorhergehenden Ansprüche von der Art, daß das genannte längliche Substrat (39) den genannten elastischen Bereich des genannten kreisförmigen Endlos-Taillenbandes umfaßt.

7. Ein wegwerfbarer absorbierender Artikel (10) nach einem der vorhergehenden Ansprüche von der Art, daß das genannte längliche Substrat ein elastisches Laminat ist, welches ein faseriges Faservlies-Substrat umfaßt.

8. Ein wegwerfbarer absorbierender Artikel (10) nach einem der vorhergehenden Ansprüche von der Art, daß der genannte elastische Bereich des genannten kreisförmigen Endlos-Taillenbandes durch thermische oder mechanische Wirkung aktiviert wird, nachdem das genannte kreisförmige Endlos-Taillenband gebildet worden ist.

9. Ein wegwerfbarer absorbierender Artikel (10) nach einem der vorhergehenden Ansprüche von der Art, daß der genannte wegwerfbare absorbierende Artikel (10) weiters zusammenwirkende Verschlußmittel umfaßt, welche gestatten, das genannte längliche Substrat (30) mit dem zweiten Längsendbereich (24) lösbar zu verbinden.

10. Ein wegwerfbarer absorbierender Artikel (10) nach Anspruch 9 von der Art, daß mindestens eines der genannten zusammenwirkenden Verschlußmittel ein Klebeband umfaßt.

11. Ein wegwerfbarer absorbierender Artikel (10) nach Anspruch 9 von der Art, daß die genannten zusammenwirkenden Verschlußmittel mechanische Befestigungsmittel umfassen.

12. Ein wegwerfbarer absorbierender Artikel (10) nach Anspruch 11 von der Art, daß die genannten mechanischen Befestigungsmittel von Haken-Schlaufen-Klettenart sind.

13. Ein wegwerfbarer absorbierender Artikel (10) nach irgendeinem der Ansprüche 9 bis 12 von der Art, daß mindestens eines der genannten zusammenwirkenden Verschlußmittel an der Innenseite des genannten kreisförmigen Endlos-Taillenbandes beigestellt ist.

14. Ein wegwerfbarer absorbierender Artikel (10) nach einem der vorhergehenden Ansprüche von der Art, daß der genannte wegwerfbare absorbierende Artikel (10) eine Windel (17) ist, welche ein flüssigkeitsdurchlässiges Deckblatt (12), ein flüssigkeitsundurchlässiges Rückenblatt (11), welches mit dem genannten Deckblatt (12) verbunden ist, und einen zwischen dem genannten Deckblatt (12) und dem genannten Rückenblatt (11) angeordneten absorbierenden Kern (14) umfaßt.

## Revendications

1. Article absorbant à jeter après usage (10) ayant une direction longitudinale (21) qui délimite une première et une seconde zone longitudinale d'extrémité (23, 24) et une direction latérale (22) qui délimite des zones latérales de côté (25) et ledit article absorbant à jeter après usage (10) comprenant :
un support oblong (30),
une bande de ceinture circulaire sans fin comprenant une zone élastique,
ledit support oblong (30) étant réuni à ladite partie de couche (17) dans chaque zone latérale de côté (25) de telle sorte que ledit support oblong (30) avec ladite partie de couche (17) forme ladite bande de ceinture circulaire sans fin ;
ladite partie élastique ayant au moins une élasticité tangentielle par rapport à ladite bande de ceinture circulaire sans fin ;
caractérisé en ce que :
ledit support oblong (30) est disposé dans ladite direction latérale (22) dans ladite première zone longitudinale d'extrémité (23) ;
ledit support oblong (30) s'étendant à la fois dans lesdites zones latérales de côté (25) de ladite partie de couche (17) et ledit support oblong (30) étant réuni audit article absorbant à jeter après usage (10) par l'intermédiaire d'une liaison adhésive ou autogène de 3 mm à 30 mm de large dans ladite direction latérale (22) sur, de préférence, 5 mm à 15 mm dans ladite direction latérale (22).

2. Article absorbant à jeter après usage (10) selon la revendication 1, dans lequel ledit support oblong (30) est réuni audit article absorbant à jeter après usage (10) le long dudit bord latéral extérieur (25) dans ladite direction longitudinale (21).

3. Article absorbant à jeter après usage (10) selon l'une quelconque des revendications précédentes, dans lequel ladite liaison autogène est formée à l'aide d'une soudure aux ultrasons ou par déformation, de préférence par sertissage.

4. Article absorbant à jeter après usage (10) selon l'une quelconque des revendications précédentes, dans lequel ledit support oblong (30) est disposé le long du bord extérieur dans la direction longitudinale (21) de ladite première zone longitudinale d'extrémité (23) dudit article absorbant à jeter après usage (10).

5. Article absorbant à jeter après usage (10) selon l'une quelconque des revendications précédentes, dans lequel ladite première zone longitudinale d'extrémité (23) est la zone d'extrémité arrière dudit article absorbant à jeter après usage (10).

6. Article absorbant à jeter après usage (10) selon l'une quelconque des revendications précédentes, dans lequel ledit support oblong (30) comporte ladite zone élastique de ladite bande de ceinture circulaire sans fin.

7. Article absorbant à jeter après usage (10) selon l'une quelconque des revendications précédentes, dans lequel ledit support oblong est un stratifié élastique comportant un support fibreux non tissé.

8. Article absorbant à jeter après usage (10) selon l'une quelconque des revendications précédentes, dans lequel ladite zone élastique de ladite bande de ceinture circulaire sans fin est activée à l'aide d'une action thermique ou mécanique une fois que ladite bande de ceinture circulaire sans fin a été formée.

9. Article absorbant à jeter après usage (10) selon l'une quelconque des revendications précédentes, dans lequel ledit article absorbant à jeter après usage (10) comporte, en outre, des moyens de fermeture coopérant permettant de réunir de façon détachable ledit support oblong (30) à la seconde zone longitudinale d'extrémité (24).

10. Article absorbant à jeter après usage (10) selon la revendication 9, dans lequel au moins l'un desdits moyens de fermeture coopérant comporte un ruban adhésif

11. Article absorbant à jeter après usage (10) selon la revendication 9, dans lequel lesdits moyens de fermeture coopérant comportent des moyens d'attache mécaniques.

12. Article absorbant à jeter après usage (10) selon la revendication 11, dans lequel lesdits moyens d'attache mécaniques sont du type à enchevêtrement par crochet-boucle.

13. Article absorbant à jeter après usage (10) selon l'une quelconque des revendications 9 à 12, dans lequel au moins l'un desdits moyens de fermeture coopérant est prévu à l'intérieur de ladite bande de ceinture circulaire sans fin.

14. Article absorbant à jeter après usage (10) selon l'une quelconque des revendications précédentes, dans lequel ledit article absorbant à jeter après usage (10) est une couche (17) comprenant une feuille de dessus perméable aux liquides (12), une feuille de fond imperméable aux liquides (11), réunie à ladite feuille de dessus (12) et une âme absorbante (14), disposée entre ladite feuille de dessus (12) et ladite feuille de fond (11).
